# EUROPEAN PATENT APPLICATION

(11) **EP 0 984 063 A2**
(43) Date of publication of application: **08.03.2000**
(21) Application number: 99305643.1
(22) Date of filing: 16.07.1999
(51) Int. Cl.: C12N 15/19, C07K 14/52, A61K 38/19, C07K 16/24

(54) **VEGF Related gene and protein**

(30) Priority: 31.08.1998 US 98548 P
(71) Applicant: ELI LILLY AND COMPANY, Indianapolis, Indiana 46285 (US)
(72) Inventor: Dou, Shenshen, Carmel, Indiana 46033 (US); Na, Songqing, Carmel, Indiana 46032 (US); Song, Ho Yeong, Carmel, Indiana 46032 (US); Su, Eric Wen, Carmel, Indiana 46032 (US); Wei, Jian-Jun, Edison, New Jersey 08820 (US)
(74) Representative: Denholm, Anna Marie

(57) **Abstract**

The invention provides isolated nucleic acid compounds, proteins and peptides, said proteins and peptides being related to the VEGF family of angiogenic ligands. Also provided are vectors and transformed heterologous host cells for expressing the proteins. Also provided are methods for identifying compounds that bind and/or modulate the activity of said proteins, methods for treating cancer or inhibiting tumor growth by administration of an antagonist of VEGF-R, and methods for stimulating angiogenesis in a patient in need thereof by administration of a therapeutically effective amount of VEGF-R.

## Description

### BACKGROUND OF THE INVENTION

This invention relates to recombinant DNA technology. In particular the invention pertains to a vascular endothelial growth factor (VEGF-R)gene, and its encoded protein. Also contemplated are methods for identifying compounds that bind said protein, and/or antagonize said protein, and methods for stimulating angiogenesis, as well as methods for inhibiting tumor growth in mammals by administration of an antagonist of said VEGF-R protein.

The growth of solid tumors and the formation of metastases are dependent on the formation of new blood vessels. Among the various angiogenic factors, VEGF plays a pivotal role in tumor angiogenesis. VEGF is expressed and secreted by most solid tumors. Moreover, VEGF is a specific mitogen for endothelial cells that induces endothelial cell migration, formation of tube-like structures, and vascular permeability. In addition, VEGF receptors are expressed preferentially in vessels lining and penetrating tumors. Thus, the family of VEGF molecules are of therapeutic interest as targets for inhibiting tumor growth. Moreover, VEGF molecules are of therapeutic interest in promoting angiogenesis, for example, as a treatment for cardiac artery blockage or for the treatment of atherosclerosis.

VEGF is a heat-stable, 46 KD dimeric protein with structural similarity to placental growth factor (PIGF) and more distant homology to the platelet-derived grwoth factors. Ferrara, N. *et.al. Endocr. Rev.* 13, 18-32, 1992. The biological activites of VEGF are mediated by two transmembrane receptor tyrosine kinases, termed flt-1 and flk-1/KDR, that are expressed predominantly on vascular endothelial cells and their embryonic progenitors.

The biological activity of VEGF and cognate molecules make them prime candidates for therapeutic development. For example, injections of VEGF protein, and DNA molecules encoding VEGF, have resulted in formation of new vessels in embryonic tissues. Drake CJ and Little CD. *Proc. Nat. Acad. Sci.* 92, 7657-661, 1995. VEGF administration may also be useful for angiogenesis *in vivo* in adult tissues through local administration to the target tissue.

### BRIEF SUMMARY OF THE INVENTION

The present invention provides isolated nucleic acid molecules that encode novel molecules related to the VEGF family, termed herein "VEGF-R". The progenitor molecule disclosed herein was identified from a human source. Having the cloned VEGF-R gene enables the production of recombinant VEGF-R protein, the isolation of orthologous genes from other organisms, and/or paralogous genes from the same organism, chromosome mapping studies, and the implementation of large scale screens to identify compounds that inhibit the binding of said VEGF-R or related molecule to its cognate receptor protein, as a means to identify potential pharmaceutical compounds useful for inhibiting tumor growth or for treating cancer. The proteins, peptides, and analogs thereof, described herein are also useful therapeutic agents, for stimulating angiogenesis in a patient in need thereof, for example, in the treatment of atherosclerosis, or cardiopathies involving blood vessel blockage, or diminished blood flow.

In one embodiment the present invention relates to VEGF-R protein (SEQ ID NO:2), and analogs thereof that are structurally and/or functionally related to SEQ ID NO:2.

In another embodiment the present invention relates to a modified, soluble VEGF-R protein comprising a carboxyl end fragment of SEQ ID NO:2.

In another embodiment the present invention relates to an isolated nucleic acid molecule encoding VEGF-R protein, and related proteins described herein.

In another embodiment, the invention relates to a nucleic acid molecule comprising the nucleotide sequence identified as SEQ ID NO:1, and/or residues 276 through 1310 of SEQ ID NO:1.

In another embodiment, the present invention relates to a nucleic acid that encodes SEQ ID NO:2, or functional fragment thereof.

In another embodiment, the present invention relates to a nucleic acid that hybridizes to a nucleic acid defined by residues 276 through 1310 of SEQ ID NO:1 under high stringency conditions, and encodes a protein that is capable of inducing angiogenesis, and/or in treating blood vessel blockage in vivo or in vitro.

In yet another embodiment, the present invention relates to a recombinant DNA vector that incorporates the VEGF-R gene (residues 276 through 1310 of SEQ ID NO:1) in operable-linkage to gene expression sequences, enabling said gene to be transcribed and translated in a host cell.

In still another embodiment the present invention relates to host cells that have been transformed or transfected with the cloned VEGF-R gene such that said gene is expressed in the host cell.

In another embodiment, the present invention relates to a method for treating cancer, and/or in preventing or inhibiting tumor growth, and/or in causing shrinkage of a cancerous tumor, *in vivo* or *in vitro* by treatment with an antagonist of VEGF-R.

In yet another embodiment, the present invention relates to a pharmaceutical formulation comprising as an active ingredient a therapeutically effective amount of an VEGF-R, associated with one or more pharmaceutically acceptable carriers, excipients, or diluents thereof.

In a further embodiment, the present invention relates to a pharmaceutical formulation comprising an VEGF-R antagonist associated with one or more pharmaceutically acceptable carriers, excipients, or diluents thereof.

In still another embodiment the present invention relates to a method for identifying an antagonist of an VEGF-R, wherein said antagonist interferes in the binding of said VEGF-R to its receptor, comprising the steps of: mixing an VEGF-R protein preparation with a test sample, or a control sample; monitoring the binding of said VEGF-R protein to its receptor, by any suitable means; and comparing the level of binding of VEGF-R to its receptor in the test sample to the level of said binding in the control sample.

### DETAILED DESCRIPTION OF THE INVENTION

### Definitions

The term "analog" or "functional analog" refers to a modified form of VEGF-R in which at least one amino acid substitution has been made such that said analog retains substantially the same biological activity as the unmodified VEGF-R in vivo and/or in vitro.

"Angiogenesis" refers to the phenomenon of blood vessel formation and differentiation in vivo and/or in vitro.

The terms "complementary" or "complementarity" as used herein refer to the capacity of purine and pyrimidine nucleotides to associate through hydrogen bonding to form double stranded nucleic acid molecules. The following base pairs are related by complementarity: guanine and cytosine; adenine and thymine; and adenine and uracil. As used herein, "complementary" means that the aforementioned relationship applies to substantially all base pairs comprising two single-stranded nucleic acid molecules over the entire length of said molecules. "Partially complementary" refers to the aforementioned relationship in which one of two single-stranded nucleic acid molecules is shorter in length than the other such that a portion of one of the molecules remains single-stranded.

The term "conservative substitution" or "conservative amino acid substitution" refers to a replacement of one or more amino acid residue(s) in a parent protein as stipulated by Table 1.

"Fragment thereof" refers to a fragment, piece, or sub-region of a nucleic acid or protein molecule whose sequence is disclosed herein, such that said fragment comprises 5 or more amino acids, or 10 or more nucleotides that are contiguous in the parent protein or nucleic acid molecule.

"Functional fragment" as used herein, refers to an isolated sub-region, or fragment of a protein disclosed herein, or sequence of amino acids that, for example, comprises a functionally distinct region such as an active site for an enzyme, or a binding site for a substrate, or a binding site for a receptor. Functional fragments may be produced by cloning technology, or as the natural products of alternative splicing mechanisms.

"Functionally related" as used herein is applied to proteins or peptides that are predicted to be functionally similar or identical to a progenitor molecule, for example, VEGF-R or fragment thereof. Functionally related species are identified based on chemical and physical similarities in amino acid composition and sequence.

"Host cell" refers to any eucaryotic or procaryotic cell that is suitable for propagating and/or expressing a cloned gene contained on a vector that is introduced into said host cell by, for example, transformation or transfection, or the like.

VEGF-R refers to a gene or cDNA (SEQ ID NO:1) and a protein (SEQ ID NO:2). VEGF-R is a member of the family of VEGF related ligands. This family of molecules is primarily responsible for angiogenesis during embryogenesis and in adult tissues, including tumor tissues.

The term "homolog" or "homologous" describes the relationship between different nucleic acid molecules or amino acid sequences in which said sequences or molecules are related by partial identity or similarity at one or more blocks or regions within said molecules or sequences.

The term "hybridization" as used herein refers to a process in which a single-stranded nucleic acid molecule joins with a complementary strand through nucleotide base pairing. "Selective hybridization" refers to hybridization under conditions of high stringency. The degree of hybridization depends upon, for example, the degree of homology, the stringency of hybridization, and the length of hybridizing strands.

"Isolated nucleic acid compound" refers to any RNA or DNA sequence, however constructed or synthesized, which is locationally distinct from its natural location.

A "nucleic acid probe" or "probe" as used herein is a labeled nucleic acid compound which hybridizes with another nucleic acid compound. "Nucleic acid probe" means a single stranded nucleic acid sequence that will combine with a complementary or partially complementary single stranded target nucleic acid sequence to form a double-stranded molecule. A nucleic acid probe may be an oligonucleotide or a nucleotide polymer. A probe will usually contain a detectable moiety which may be attached to the end(s) of the probe or be internal to the sequence of the probe.

The term "orthologue" or "orthologous" refers to two or more genes or proteins from different organisms that exhibit sequence homology.

The term "paralogue" or "paralogous" refers to two or more genes or proteins within a single organism that exhibit sequence homology.

The term "plasmid" refers to an extrachromosomal genetic element. The plasmids disclosed herein are commercially available, publicly available on an unrestricted basis, or can be constructed from readily available plasmids in accordance with published procedures.

A "primer" is a nucleic acid fragment which functions as an initiating substrate for enzymatic or synthetic elongation of, for example, a nucleic acid molecule.

The term "promoter" refers to a nucleic acid sequence that directs transcription, for example, of DNA to RNA. An inducible promoter is one that is regulatable by environmental signals, such as carbon source, heat, or metal ions, for example. A constitutive promoter generally operates at a constant level and is not regulatable.

"Recombinant DNA cloning vector" as used herein refers to any autonomously replicating agent, including, but not limited to, plasmids and phages, comprising a DNA molecule to which one or more additional DNA segments can or have been incorporated.

The term "recombinant DNA expression vector" or "expression vector" as used herein refers to any recombinant DNA cloning vector, for example a plasmid or phage, in which a promoter and other regulatory elements are present thereby enabling transcription of an inserted DNA, which may encode a protein.

The term "stringency" refers to hybridization conditions. High stringency conditions disfavor non-homologous base pairing. Low stringency conditions have the opposite effect. Stringency may be altered, for example, by temperature and salt concentration.

"Low stringency" conditions comprise, for example, a temperature of about 37° C or less, a formamide concentration of less than about 50%, and a moderate to low salt (SSC) concentration; or, alternatively, a temperature of about 50° C or less, and a moderate to high salt (SSPE) concentration, for example 1M NaCl.

"High stringency" conditions comprise, for example, a temperature of about 42° C or less, a formamide concentration of less than about 20%, and a low salt (SSC) concentration; or, alternatively, a temperature of about 65° C, or less, and a low salt (SSPE) concentration. For example, high stringency conditions comprise hybridization in 0.5 M NaHPO₄, 7% sodium dodecyl sulfate (SDS), 1 mM EDTA at 65°C (Ausubel, F.M. *et al.* Current Protocols in Molecular Biology, Vol. I, 1989; Green Inc. New York, at 2.10.3).

"SSC" comprises a hybridization and wash solution. A stock 20X SSC solution contains 3M sodium chloride, 0.3M sodium citrate, pH 7.0.

"SSPE" comprises a hybridization and wash solution. A 1X SSPE solution contains 180 mM NaCl, 9mM Na₂HPO₄, 0.9 mM NaH₂PO₄ and 1 mM EDTA, pH 7.4.

"Substantially pure," used in reference to a peptide or protein, means separation from other cellular and non-cellular molecules, including other protein molecules. A substantially pure preparation would be about at least 85% pure; preferably about at least 95% pure. A "substantially pure" protein can be prepared by a variety of techniques, well known to the skilled artisan, including, for example, the IMAC protein purification method.

"Treating" as used herein describes the management and care of a patient for the purpose of combating the disease, condition, or disorder and includes the administration of a protein of the present invention to prevent the onset of the symptoms or complications, alleviating the symptoms or complications, or eliminating the disease, condition, or disorder. Treating as used herein includes the administration of the protein for cosmetic purposes. A cosmetic purpose seeks to control, for example, the weight of a mammal to improve bodily appearance.

The term "vector" as used herein refers to a nucleic acid compound used for introducing exogenous or endogenous DNA into host cells. A vector comprises a nucleotide sequence which may encode one or more protein molecules. Plasmids, cosmids, viruses, and bacteriophages, in the natural state or which have undergone recombinant engineering, are examples of commonly used vectors.

The various restriction enzymes disclosed and described herein are commercially available and the manner of use of said enzymes including reaction conditions, cofactors, and other requirements for activity are well known to one of ordinary skill in the art. Reaction conditions for particular enzymes were carried out according to the manufacturer's recommendation.

The VEGF-R gene encodes a novel protein that is related to the VEGF family of proteins. The VEGF-R cDNA comprises a DNA sequence specified herein by SEQ ID NO:1, the coding region being defined by residues 276 through 1310 of SEQ ID NO:1. Those skilled in the art will recognize that owing to the degeneracy of the genetic code, numerous "silent" substitutions of nucleotide base pairs could be introduced into the sequence identified as SEQ ID NO:1 without altering the identity of the encoded amino acid(s) or protein product. All such substitutions are intended to be within the scope of the invention.

Also contemplated by the present invention are VEGF-R proteins, functional fragments thereof, analogs, and functionally related molecules. The VEGF-R protein may be membrane-bound in vivo. For example, expression of a VEGF-R DNA expression vector in 293 T cells or CHO cells showed VEGF-R on the cell membrane of transfected cells as detected by FACS staining using an anti-VEGF-R serum. A secreted form of VEGF-R can be produced by removing a portion of the sequence at the amino terminus and fusing therefore, any suitable signal peptide to facilitate secretion from an expression host cell. For example, residues from about 1 through about 22 of SEQ ID NO:2, or residues from about 1 through 15 of SEQ ID NO:2 can be removed and replaced with the kappa light chain signal sequence, or any other suitable signal peptide, for example the protrypsin signal peptide. Functional fragments comprising sub-regions of VEGF-R (SEQ ID NO:2) are also contemplated by the present invention. One functional fragment comprises from about residues 1 through about 29 of SEQ ID NO:2; another functional fragment comprises from about residues 30 through about 80 of SEQ ID NO:2. These subregions define a kinase domain receptor (KDR) binding site.

Other functional fragments are conveniently identified as fragments of an intact VEGF-R protein that retain the capacity to induce angiogenesis in vivo or in vitro.

Amino acid substitution modifications can be made in accordance with the following Table. Modifications of VEGF-R peptides made in accordance with the Table are generally expected to retain the biological activity of the parent molecule based on the art recognized substitutability of certain amino acids (*See e.g.* M. Dayhoff, In Atlas of Protein Sequence and Structure, Vol. 5, Supp. 3, pgs 345-352, 1978). Functionality is easily tested in an assay that measures endothelial cell mitogenic activity, for example.

| ORIGINAL RESIDUE | EXEMPLARY SUBSTITUTIONS |
|---|---|
| ALA | SER, THR |
| ARG | LYS |
| ASN | HIS, SER |
| ASP | GLU, ASN |
| CYS | SER |
| GLN | ASN, HIS |
| GLU | ASP, GLU |
| GLY | ALA, SER |
| HIS | ASN, GLN |
| ILE | LEU, VAL, THR |
| LEU | ILE, VAL |
| LYS | ARG, GLN, GLU, THR |
| MET | LEU, ILE, VAL |
| PHE | LEU, TYR |
| SER | THR, ALA, ASN |
| THR | SER, ALA |
| TRP | ARG, SER |
| TYR | PHE |
| VAL | ILE, LEU, ALA |
| PRO | ALA |

### Functionally related proteins and peptides

Analogs having biological activities that are similar or identical to VEGF-R, for example, the ability to induce angiogenesis, in vivo or in vitro, are also contemplated by the present invention. Said analogs, while being functionally related, comprise amino acid sequences that differ from SEQ ID NO:2. Functional analogs of VEGF-R can be generated by deletion, insertion, inversion, and/or substitution of one or more amino acid residues in said VEGF-R. Substitution analogs can generally be made by solid phase or recombinant techniques in which single or multiple conservative amino acid substitutions are made, for example, according to Table 1. Generally, in the case of multiple substitutions, it is preferred that less than ten residues be changed in any given molecule, most preferably between one to five residues are changed in any given molecule such that about between 90% to 99% of residues are identical with the sequence of SEQ ID NO:2; alternatively, such that about between 95% to 99% of residues are identical with SEQ ID NO:2.

### Fragments of proteins

One embodiment of the instant invention provides fragments of the proteins disclosed that may or may not be biologically active. Such fragments are useful, for example, as an antigen for producing an antibody to said proteins.

Fragments of the proteins disclosed herein may be generated by any number of suitable techniques, including chemical synthesis of any portion of SEQ ID NO:2, proteolytic digestion of SEQ ID NO:2, or most preferably, by recombinant DNA mutagenesis techniques, well known to the skilled artisan. *See. e.g.* K. Struhl, "Reverse biochemistry: Methods and applications for synthesizing yeast proteins *in vitro," Meth. Enzymol.* 194, 520-535. For example, in a preferred method, a nested set of deletion mutations are introduced into a nucleic acid sequence encoding VEGF-R (e.g. residues 276 through 1310 of SEQ ID NO:1) such that varying amounts of the protein coding region are deleted, either from the amino terminal end, or from the carboxyl end of the protein molecule. This method can also be used to create internal fragments of the intact protein in which both the carboxyl and amino terminal ends are removed. Several appropriate nucleases can be used to create such deletions, for example *Bal*31, or in the case of a single stranded nucleic acid molecule, mung bean nuclease. For simplicity, it is preferred that the VEGF-R gene be cloned into a single-stranded cloning vector, such as bacteriophage M13, or equivalent. If desired, the resulting gene deletion fragments can be subcloned into any suitable vector for propagation and expression of said fragments in any suitable host cell.

Functional fragments of the proteins disclosed herein may be produced as described above, preferably using cloning techniques to engineer smaller versions of the intact gene, lacking sequence from the 5' end, the 3' end, from both ends, or from an internal site. Fragments may be tested for biological activity using any suitable assay, for example, the ability of a protein fragment to induce apoptosis, *in vivo* or *in vitro*.

### Gene Isolation Procedures

Those skilled in the art will recognize that the VEGF-R gene could be obtained by a plurality of recombinant DNA techniques including, for example, hybridization, polymerase chain reaction (PCR) amplification, or de novo DNA synthesis. (*See e.g.*, T. Maniatis *et al*. Molecular Cloning: A Laboratory Manual, 2d Ed. Chap. 14 (1989)).

Methods for constructing cDNA libraries in a suitable vector such as a plasmid or phage for propagation in procaryotic or eucaryotic cells are well known to those skilled in the art. [*See e.g.* Maniatis *et al. Supra*]. Suitable cloning vectors are well known and are widely available.

The VEGF-R gene, or fragment thereof, can be isolated from a tissue in which said gene is expressed, for example, placenta. In one method, mRNA is isolated, and first strand cDNA synthesis is carried out. A second round of DNA synthesis can be carried out for the production of the second strand. If desired, the double-stranded cDNA can be cloned into any suitable vector, for example, a plasmid, thereby forming a cDNA library. Oligonucleotide primers targeted to any suitable region of SEQ ID NO:1 can be used for PCR amplification of VEGF-R. *See e.g.* PCR Protocols: A Guide to Method and Application, Ed. M. Innis et al., Academic Press (1990). The PCR amplification comprises template DNA, suitable enzymes, primers, and buffers, and is conveniently carried out in a DNA Thermal Cycler (Perkin Elmer Cetus, Norwalk, CT). A positive result is determined by detecting an appropriately-sized DNA fragment following agarose gel electrophoresis.

### Protein Production Methods

One embodiment of the present invention relates to the substantially purified protein encoded by the VEGF-R gene.

Skilled artisans will recognize that the proteins of the present invention can be synthesized by a number of different methods, such as chemical methods well known in the art, including solid phase peptide synthesis or recombinant methods. Both methods are described in U.S. Patent 4,617,149, incorporated herein by reference.

The principles of solid phase chemical synthesis of polypeptides are well known in the art and may be found in general texts in the area. *See, e.g.*, H. Dugas and C. Penney, Bioorganic Chemistry (1981) Springer-Verlag, New York, 54-92. For example, peptides may be synthesized by solid-phase methodology utilizing an Applied Biosystems 430A peptide synthesizer (Applied Biosystems, Foster City, CA) and synthesis cycles supplied by Applied Biosystems.

The proteins of the present invention can also be produced by recombinant DNA methods using the cloned VEGF-R gene. Recombinant methods are preferred if a high yield is desired. Expression of the cloned gene can be carried out in a variety of suitable host cells, well known to those skilled in the art. For this purpose, the VEGF-R gene is introduced into a host cell by any suitable means, well known to those skilled in the art. While chromosomal integration of the cloned gene is within the scope of the present invention, it is preferred that the gene be cloned into a suitable extra-chromosomally maintained expression vector so that the coding region of the VEGF-R gene is operably-linked to a constitutive or inducible promoter.

The basic steps in the recombinant production of the VEGF-R protein are:
a) constructing a natural, synthetic or semi-synthetic DNA encoding VEGF-R protein;
b) integrating said DNA into an expression vector in a manner suitable for expressing the VEGF-R protein, either alone or as a fusion protein;
c) transforming or otherwise introducing said vector into an appropriate eucaryotic or prokaryotic host cell forming a recombinant host cell,
d) culturing said recombinant host cell in a manner to express the VEGF-R protein; and
e) recovering and substantially purifying the VEGF-R protein by any suitable means, well known to those skilled in the art.

### Expressing Recombinant VEGF-R Protein in Procaryotic and Eucaryotic Host Cells

Procaryotes may be employed in the production of recombinant VEGF-R protein. For example, the *Escherichia coli* K12 strain 294 (ATCC No. 31446) is particularly useful for the prokaryotic expression of foreign proteins. Other strains of *E. coli*, bacilli such as *Bacillus subtilis*, enterobacteriaceae such as *Salmonella typhimurium* or *Serratia marcescans*, various Pseudomonas species and other bacteria, such as *Streptomyces*, may also be employed as host cells in the cloning and expression of the recombinant proteins of this invention.

Promoter sequences suitable for driving the expression of genes in procaryotes include b -lactamase [*e.g.* vector pGX2907, ATCC 39344, contains a replicon and b -lactamase gene], lactose systems [Chang et al., *Nature* (London), 275:615 (1978); Goeddel et al., *Nature* (London), 281:544 (1979)], alkaline phosphatase, and the tryptophan (trp) promoter system [vector pATH1 (ATCC 37695)], which is designed to facilitate expression of an open reading frame as a trpE fusion protein under the control of the trp promoter. Hybrid promoters such as the tac promoter (isolatable from plasmid pDR540, ATCC-37282) are also suitable. Still other bacterial promoters, whose nucleotide sequences are generally known, may be ligated to DNA encoding the protein of the instant invention, using linkers or adapters to supply any required restriction sites. Promoters for use in bacterial systems also will contain a Shine-Dalgarno sequence operably-linked to the DNA encoding the desired polypeptides. These examples are illustrative rather than limiting.

The proteins of this invention may be synthesized either by direct expression or as a fusion protein comprising the protein of interest as a translational fusion with another protein or peptide which may be removable by enzymatic or chemical cleavage. It is often observed in the production of certain peptides in recombinant systems that expression as a fusion protein prolongs the life span, increases the yield of the desired peptide, or provides a convenient means of purifying the protein. This is particularly relevant when expressing mammalian proteins in procaryotic hosts. A variety of peptidases (e.g. enterokinase and thrombin) which cleave a polypeptide at specific sites or digest the peptides from the amino or carboxy termini (*e.g.* diaminopeptidase) of the peptide chain are known. Furthermore, particular chemicals (*e.g.* cyanogen bromide) will cleave a polypeptide chain at specific sites. The skilled artisan will appreciate the modifications necessary to the amino acid sequence (and synthetic or semi-synthetic coding sequence if recombinant means are employed) to incorporate site-specific internal cleavage sites. *See e.g.*, P. Carter, "Site Specific Proteolysis of Fusion Proteins", Chapter 13, in Protein Purification: From Molecular Mechanisms to Large Scale Processes, American Chemical Society, Washington, D.C. (1990).

In addition to procaryotes, a variety of amphibian expression systems such as frog oocytes, and mammalian cell systems can be used. The choice of a particular host cell depends to some extent on the particular expression vector used. Exemplary mammalian host cells suitable for use in the present invention include HepG-2 (ATCC HB 8065), CV-1 (ATCC CCL 70), LC-MK₂ (ATCC CCL 7.1), 3T3 (ATCC CCL 92), CHO-K1 (ATCC CCL 61), HeLa (ATCC CCL 2), RPMI8226 (ATCC CCL 155), H4IIEC3 (ATCC CCL 1600), C127I (ATCC CCL 1616), HS-Sultan (ATCC CCL 1484), and BHK-21 (ATCC CCL 10), for example.

A wide variety of vectors are suitable for transforming mammalian host cells. For example, the pSV2-type vectors comprise segments of the simian virus 40 (SV40) genome required for transcription and polyadenylation. A large number of plasmid pSV2-type vectors have been constructed, such as pSV2-gpt, pSV2-neo, pSV2-dhfr, pSV2-hyg, and pSV2-b-globin, in which the SV40 promoter drives transcription of an inserted gene. These vectors are widely available from sources such as the American Type Culture Collection (ATCC), 12301 Parklawn Drive, Rockville, Maryland, 20852, or the Northern Regional Research Laboratory (NRRL), 1815 N. University Street, Peoria, Illinois, 61604.

Promoters suitable for expression in mammalian cells include the SV40 late promoter, promoters from eukaryotic genes, such as, for example, the estrogen-inducible chicken ovalbumin gene, the interferon genes, the glucocorticoid-inducible tyrosine aminotransferase gene, the thymidine kinase gene promoter, and the promoters of the major early and late adenovirus genes and the cytomegalovirus promoter.

Plasmid pRSVcat (ATCC 37152) comprises portions of a long terminal repeat of the Rous Sarcoma virus, a virus known to infect chickens and other host cells. This long terminal repeat contains a promoter which is suitable for use in the vectors of this invention. H. Gorman *et al.*, *Proc. Nat. Acad. Sci. (USA)*, 79, 6777 (1982). The plasmid pMSVi (NRRL B-15929) comprises the long terminal repeats of the Murine Sarcoma virus, a virus known to infect mouse and other host cells. The mouse metallothionein promoter has also been well characterized for use in eukaryotic host cells and is suitable for use in the present invention. This promoter is present in the plasmid pdBPV-MMTneo (ATCC 37224) which can serve as the starting material for the construction of other plasmids of the present invention.

Transfection of mammalian cells with vectors can be performed by a plurality of well known processes including, but not limited to, protoplast fusion, calcium phosphate co-precipitation, electroporation and the like. *See, e.g.*, Maniatis *et al., supra*.

Some viruses also make appropriate vectors. Examples include the adenoviruses, the adeno-associated viruses, the vaccinia virus, the herpes viruses, the baculoviruses, and the rous sarcoma virus, as described in U.S. Patent 4,775,624, incorporated herein by reference.

Eucaryotic microorganisms such as yeast and other fungi are also suitable host cells. The yeast *Saccharomyces cerevisiae* is the preferred eucaryotic microorganism. Other yeasts such as *Kluyveromyces lactis* and *Pichia pastoris* are also suitable. For expression in *Saccharomyces*, the plasmid YRp7 (ATCC-40053), for example, may be used. *See, e.g.*, L. Stinchcomb *et al., Nature*, 282, 39 (1979); J. Kingsman *et al., Gene*, 7, 141 (1979); S. Tschemper *et al., Gene*, 10, 157 (1980). Plasmid YRp7 contains the TRP1 gene which provides a selectable marker for use in a trp1 auxotrophic mutant.

### Purification of Recombinantly-Produced VEGF-R Protein

An expression vector carrying the cloned VEGF-R gene is transformed or transfected into a suitable host cell using standard methods. Cells that contain the vector are propagated under conditions suitable for expression of the recombinant VEGF-R protein. For Example, if the recombinant gene has been placed under the control of an inducible promoter, suitable growth conditions would incorporate the appropriate inducer. The recombinantly-produced protein may be purified from cellular extracts of transformed cells by any suitable means.

In a preferred process for protein purification, the VEGF-R gene is modified at the 5' end to incorporate several histidine residues at the amino terminus of the VEGF-R protein. This "histidine tag" enables a single-step protein purification method referred to as "immobilized metal ion affinity chromatography" (IMAC), essentially as described in U.S. Patent 4,569,794, which hereby is incorporated by reference. The IMAC method enables rapid isolation of substantially pure recombinant VEGF-R protein starting from a crude extract of cells that express a modified recombinant protein, as described above.

### Production of Antibodies

The proteins of this invention and fragments thereof may be used in the production of antibodies. The term "antibody" as used herein describes antibodies, fragments of antibodies (such as, but not limited, to Fab, Fab', Fab₂', and Fv fragments), and chimeric, humanized, veneered, resurfaced, or CDR-grafted antibodies capable of binding antigens of a similar nature as the parent antibody molecule from which they are derived. The instant invention also encompasses single chain polypeptide binding molecules.

The production of antibodies, both monoclonal and polyclonal, in animals, especially mice, is well known in the art. *See, e.g.*, C. Milstein, Handbook of Experimental Immunology, (Blackwell Scientific Pub., 1986); J. Goding, Monoclonal Antibodies: Principles and Practice, (Academic Press, 1983). For the production of monoclonal antibodies the basic process begins with injecting a mouse, or other suitable animal, with an immunogen. The mouse is subsequently sacrificed and cells taken from its spleen are fused with myeloma cells, resulting in a hybridoma that reproduces *in vitro*. The population of hybridomas is screened to isolate individual clones, each of which secretes a single antibody species, specific for the immunogen. Each antibody obtained in this way is the clonal product of a single B cell.

Chimeric antibodies are described in U.S. Patent No. 4,816,567, the entire contents of which is herein incorporated by reference. This reference discloses methods and vectors for the preparation of chimeric antibodies. An alternative approach is provided in U.S. Patent No. 4,816,397, the entire contents of which is herein incorporated by reference. This patent teaches co-expression of the heavy and light chains of an antibody in the same host cell.

The approach of U.S. Patent 4,816,397 has been further refined in European Patent Publication No. 0 239 400. The teachings of this European patent publication are a preferred format for genetic engineering of monoclonal antibodies. In this technology the complementarity determining regions (CDRs) of a human antibody are replaced with the CDRs of a murine monoclonal antibody, thereby converting the specificity of the human antibody to the specificity of a murine antibody.

Single chain antibodies and libraries thereof are yet another variety of genetically engineered antibody technology that is well known in the art. (*See, e.g.* R.E. Bird, *et al., Science* 242:423-426 (1988); PCT Publication Nos. WO 88/01649, WO 90/14430, and WO 91/10737. Single chain antibody technology involves covalently joining the binding regions of heavy and light chains to generate a single polypeptide chain. The binding specificity of the intact antibody molecule is thereby reproduced on a single polypeptide chain.

The antibodies contemplated by this invention are useful in diagnostics, therapeutics or in diagnostic/therapeutic combinations.

The proteins of this invention or suitable fragments thereof can be used to generate polyclonal or monoclonal antibodies, and various inter-species hybrids, or humanized antibodies, or antibody fragments, or single-chain antibodies. The techniques for producing antibodies are well known to skilled artisans. (*See e.g.* A.M. Campbell, Monoclonal Antibody Technology: Laboratory Techniques in Biochemsitry and Molecular Biology, Elsevier Science Publishers, Amsterdam (1984); Kohler and Milstein, *Nature* 256, 495-497 (1975); Monoclonal Antibodies: Principles & Applications Ed. J.R.Birch & E.S. Lennox, Wiley-Liss, 1995.

A protein used as an immunogen may be modified or administered in an adjuvant, by subcutaneous or intraperitoneal injection into, for example, a mouse or a rabbit. For the production of monoclonal antibodies, spleen cells from immunized animals are removed, fused with myeloma cells, such as SP2/0-Ag14 cells, and allowed to become monoclonal antibody producing hybridoma cells in the manner known to the skilled artisan. Hybridomas that secrete a desired antibody molecule can be screened by a variety of well known methods, for example ELISA assay, western blot analysis, or radioimmunoassay (Lutz *et al. Exp. Cell Res.* 175, 109-124 (1988); Monoclonal Antibodies: Principles & Applications Ed. J.R.Birch & E.S. Lennox, Wiley-Liss, 1995).

For some applications labeled antibodies are desirable. Procedures for labeling antibody molecules are widely known, including for example, the use of radioisotopes, affinity labels, such as biotin or avidin, enzymatic labels, for example horseradish peroxidase, and fluorescent labels, such as FITC or rhodamine (*See e.g.* Enzyme-Mediated Immunoassay, Ed. T. Ngo, H. Lenhoff, Plenum Press 1985; Principles of Immunology and Immunodiagnostics, R.M. Aloisi, Lea & Febiger, 1988).

Labeled antibodies are useful for a variety of diagnostic applications. In one embodiment the present invention relates to the use of labeled antibodies to detect the presence of VEGF-R. Alternatively, the antibodies could be used in a screen to identify potential modulators of VEGF-R. For example, in a competitive displacement assay, the antibody or compound to be tested is labeled by any suitable method. Competitive displacement of an antibody from an antibody-antigen complex by a test compound such that a test compound-antigen complex is formed provides a method for identifying compounds that bind VEGF-R.

Other embodiments of the present invention comprise isolated nucleic acid sequences that encode SEQ ID NO:2, or related nucleic acids that are at least about 75% identical to SEQ ID NO:1, or to their complementary sequence, or nucleic acids that hybridize to SEQ ID NO:1 under high stringency conditions. Such sequences may come, for example, from paralogous or orthologous genes.

The VEGF-R cDNA (viz. SEQ ID NO:1) and related nucleic acid molecules that encode SEQ ID NO:2, or functional fragments thereof, may be produced by chemical synthetic methods. The synthesis of nucleic acids is well known in the art. *See, e.g.*, E.L. Brown, R. Belagaje, M.J. Ryan, and H.G. Khorana, *Methods in Enzymology*, 68:109-151 (1979). Fragments of the DNA sequence corresponding to the VEGF-R gene could be generated using a conventional DNA synthesizing apparatus, such as the Applied Biosystems Model 380A or 380B DNA synthesizers (Applied Biosystems, Inc., 850 Lincoln Center Drive, Foster City, CA 94404) using phosphoramidite chemistry, thereafter ligating the fragments so as to reconstitute the entire gene. Alternatively, phosphotriester chemistry may be employed to synthesize the nucleic acids of this invention. (*See*, *e.g.*, M.J. Gait, ed., Oligonucleotide Synthesis, A Practical Approach, (1984)).

In an alternative methodology, namely PCR, the DNA sequences disclosed and described herein, comprising, for example, a portion or all of SEQ ID NO:1 can be produced from a plurality of starting materials. For example, starting with a cDNA preparation (e.g. cDNA library) derived from a tissue that expresses the VEGF-R gene, suitable oligonucleotide primers complementary to SEQ ID NO:1 or to any sub-region therein, are prepared as described in U.S. Patent No. 4,889,818, hereby incorporated by reference. Other suitable protocols for the PCR are disclosed in PCR Protocols: A Guide to Method and Applications, Ed. Michael A. Innis et al., Academic Press, Inc. (1990). Using PCR, any region of the VEGF-R gene can be targeted for amplification such that full or partial length gene sequences may be produced.

The ribonucleic acids of the present invention may be prepared using polynucleotide synthetic methods discussed *supra*, or they may be prepared enzymatically, for example, using RNA polymerase to transcribe a VEGF-R DNA template.

The most preferred systems for preparing the ribonucleic acids of the present invention employ the RNA polymerase from the bacteriophage T7 or the bacteriophage SP6. These RNA polymerases are highly specific, requiring the insertion of bacteriophage-specific sequences at the 5' end of the template to be transcribed. *See*, Maniatis *et al., supra*.

This invention also provides nucleic acids, RNA or DNA, that are complementary to SEQ ID NO:1, or fragment thereof.

### Nucleic Acid Probes

The present invention also provides probes and primers useful for a variety of molecular biology techniques including, for example, hybridization screens of genomic, subgenomic, or cDNA libraries, as well as hybridization against nucleic acids derived from cell lines or tissues that originate from drug-resistant tumors. Such hybridization screens are useful as methods to identify homologous and/or functionally related sequences from the same or other organisms. A nucleic acid compound comprising SEQ ID NO:1, or a complementary sequence thereof, or a fragment thereof, which is at least 14 base pairs in length, and which will selectively hybridize to human DNA or mRNA encoding VEGF-R protein or fragment thereof, or a functionally related protein, is provided. Preferably, the 14 or more base pair compound is DNA. *See e.g.* B. Wallace and G. Miyada, "Oligonucleotide Probes for the Screening of Recombinant DNA Libraries," In *Meth. Enzym.*, 152, 432-442, Academic Press (1987).

Probes and primers can be prepared by enzymatic or recombinant methods, well known to those skilled in the art (*See e.g.* Sambrook et al. *supra*). A probe may be a single stranded nucleic acid sequence which is complementary in some particular degree to a nucleic acid sequence sought to be detected ("target sequence"). A probe may be labeled with a detectable moiety such as a radio-isotope, antigen, or chemiluminescent moiety. A description of the use of nucleic acid hybridization as a procedure for the detection of particular nucleic acid sequences is described in U.S. Patent No. 4,851,330 to Kohne, entitled "Method for Detection, Identification and Quantitation of Non-Viral Organisms."

Having the DNA sequence of the present invention allows preparation of relatively short DNA (or RNA) sequences having the ability to specifically hybridize to gene sequences disclosed herein. In these aspects, nucleic acid probes of an appropriate length are prepared. The ability of such nucleic acid probes to specifically hybridize to a polynucleotide encoding a VEGF-R gene or related sequence lends particular utility in a variety of embodiments. Most importantly, the probes may be used in a variety of assays for detecting the presence of complementary sequences in a given sample.

In certain embodiments, it is advantageous to use oligonucleotide primers. The sequence of such primers is designed using a polynucleotide of the present invention for use in detecting, amplifying or mutating a defined segment of a gene or polynucleotide that encodes a VEGF-R polypeptide using PCR technology.

Preferred nucleic acid sequences employed for hybridization studies, or assays, include probe molecules that are complementary to at least an about 14 to an about 70-nucleotide long stretch of a polynucleotide that encodes a VEGF-R polypeptide, such as the nucleotide base sequences designated as SEQ ID NO:1. A length of at least 14 nucleotides helps to ensure that the fragment is of sufficient length to form a duplex molecule that is both stable and selective. Molecules having complementary sequences over stretches greater than 14 bases in length are generally preferred, though in order to increase stability and selectivity of the hybrid. One will generally prefer to design nucleic acid molecules having gene-complementary stretches of 25 to 40 nucleotides, 55 to 70 nucleotides, or even longer where desired. Such fragments may be readily prepared by, for example, directly synthesizing the fragment by chemical means, by application of nucleic acid reproduction technology, such as the PCR TM technology of U.S. Pat. No. 4,603,102, herein incorporated by reference, or by excising selected DNA fragments from recombinant plasmids containing appropriate inserts and suitable restriction enzyme sites.

The following guidelines are useful for designing probes with desirable characteristics. The extent and specificity of hybridization reactions are affected by a number of factors that determine the sensitivity and specificity of a particular probe, whether perfectly complementary to its target or not. The affect of various experimental parameters and conditions are well known to those skilled in the art.

First, the stability of the probe:target nucleic acid hybrid should be chosen to be compatible with the assay conditions. This may be accomplished by avoiding long A and T rich sequences, by terminating the hybrids with G:C base pairs and by designing a probe with an appropriate Tm (i.e. melting temperature). The melting profile, including the Tm of a hybrid comprising an oligonucleotide and target sequence, may be determined using a Hybridization Protection Assay. The probe should be chosen so that the length and % GC content result in a Tm about 2°-10° C higher than the temperature at which the final assay will be performed. The base composition of the probe is also a significant factor because G-C base pairs exhibit greater thermal stability as compared to A-T base pairs. Thus, hybridization involving complementary nucleic acids of higher G-C content will be more stable at higher temperatures.

The ionic strength and incubation temperature under which a probe will be used should also be taken into account. It is known that hybridization will increase as the ionic strength of the reaction mixture increases, and that the thermal stability of molecular hybrids will increase with increasing ionic strength. On the other hand, chemical reagents such as formamide, urea, DMSO and alcohols, which disrupt hydrogen bonds, increase the stringency of hybridization. Destabilization of hydrogen bonds by such reagents can greatly reduce the Tm. In general, optimal hybridization for synthetic oligonucleotide probes of about 10-50 bases in length occurs approximately 5° C below the melting temperature for a given duplex. Incubation at temperatures below the optimum may allow mismatched base sequences to hybridize and can therefore result in reduced specificity.

The length of the target nucleic acid sequence and, accordingly, the length of the probe sequence can also be important. In some cases, there may be several sequences from a particular region, varying in location and length, which will yield probes with the desired hybridization characteristics. In other cases, one sequence may be significantly better than another even though the one sequence differs merely by a single base. Finally, there can be intramolecular and intermolecular hybrids formed within a probe if there is sufficient self-complementarity. Such structures can be avoided through careful probe design. Computer programs are available to search for this type of interaction.

A probe molecule may be used for hybridizing to a sample suspected of possessing a VEGF-R or VEGF-R-related nucleotide sequence. The hybridization reaction is carried out under suitable conditions of stringency.

Alternatively, such DNA molecules may be used in a number of techniques including their use as: (1) diagnostic tools to detect polymorphisms in DNA samples from a human or other mammal; (2) means for detecting and isolating homologs of VEGF-R and related polypeptides from a DNA library potentially containing such sequences; (3) primers for hybridizing to related sequences for the purpose of amplifying those sequences; and (4) primers for altering the native VEGF-R DNA sequences; as well as other techniques which rely on the similarity of the DNA sequences to those of the VEGF-R DNA segments herein disclosed.

Once synthesized, oligonucleotide probes may be labeled by any of several well known methods. *See e.g.* Maniatis *et.al.*, Molecular Cloning (2d ed. 1989). Useful labels include radioisotopes, as well as non-radioactive reporting groups. Isotopic labels include H³, S³⁵, P³², I¹²⁵, Cobalt, and C¹⁴. Most methods of isotopic labeling involve the use of enzymes and include methods such as nick-translation, end-labeling, second strand synthesis, and reverse transcription. When using radio-labeled probes, hybridization can be detected by autoradiography, scintillation counting, or gamma counting. The detection method selected will depend upon the hybridization conditions and the particular radio isotope used for labeling.

Non-isotopic materials can also be used for labeling, and may be introduced internally into the sequence or at the end of the sequence. Modified nucleotides may be incorporated enzymatically or chemically, and chemical modifications of the probe may be performed during or after synthesis of the probe, for example, by the use of non-nucleotide linker groups. Non-isotopic labels include fluorescent molecules, chemiluminescent molecules, enzymes, cofactors, enzyme substrates, haptens or other ligands. In a preferred embodiment of the invention, the length of an oligonucleotide probe is greater than or equal to about 18 nucleotides and less than or equal to about 50 nucleotides. Labeling of an oligonucleotide of the present invention may be performed enzymatically using [³²P]-labeled ATP and the enzyme T4 polynucleotide kinase.

### Vectors

Another aspect of the present invention relates to recombinant DNA cloning vectors and expression vectors comprising the nucleic acids of the present invention. The preferred nucleic acid vectors are those which comprise DNA. The most preferred recombinant DNA vectors comprise the isolated DNA sequence, defined by residues 276 through 1310 of SEQ ID NO:1.

The skilled artisan understands that choosing the most appropriate cloning vector or expression vector depends upon a number of factors including the availability of restriction enzyme sites, the type of host cell into which the vector is to be transfected or transformed, the purpose of the transfection or transformation (e.g., stable transformation as an extrachromosomal element, or integration into the host chromosome), the presence or absence of readily assayable or selectable markers (e.g., antibiotic resistance and metabolic markers of one type and another), and the number of copies of the gene desired in the host cell.

Vectors suitable to carry the nucleic acids of the present invention comprise RNA viruses, DNA viruses, lytic bacteriophages, lysogenic bacteriophages, stable bacteriophages, plasmids, viroids, and the like. The most preferred vectors are plasmids.

When preparing an expression vector the skilled artisan understands that there are many variables to be considered, for example, whether to use a constitutive or inducible promoter. The practitioner also understands that the amount of nucleic acid or protein to be produced dictates, in part, the selection of the expression system. Regarding promoter sequences, inducible promoters are preferred because they enable high level, regulatable expression of an operably-linked gene. The skilled artisan will recognize a number of suitable promoters that respond to a variety of inducers, for example, carbon source, metal ions, and heat. Other relevant considerations regarding an expression vector include whether to include sequences for directing the localization of a recombinant protein. For example, a sequence encoding a signal peptide preceding the coding region of a gene is useful for directing the extra-cellular export of a resulting polypeptide.

The present invention also provides a method for constructing a recombinant host cell capable of expressing VEGF-R proteins (e.g. SEQ ID NO:2), said method comprising transforming or otherwise introducing into a host cell a recombinant DNA vector that comprises an isolated DNA sequence that encodes SEQ ID NO:2. A suitable host cell is any eucaryotic cell that can accomodate high level expression of an exogenously introduced gene or protein, and that will incorporate said protein into its membrane structure. Vectors for expression are those which comprise SEQ ID NO:1 or fragment thereof. Transformed host cells may be cultured under conditions well known to skilled artisans such that SEQ ID NO:2 is expressed, thereby producing a recombinant VEGF-R protein in the recombinant host cell.

For the purpose of identifying compounds having utility as inhibitors of tumor growth, for example, in a treatment of cancer, it would be desirable to identify compounds that bind the VEGF-R protein and/or modify or antagonize its activity. A method for determining agents that bind the VEGF-R protein comprises contacting the VEGF-R protein with a test compound and monitoring binding by any suitable means.

The instant invention provides a screening system for discovering compounds that bind the VEGF-R protein, said screening system comprising the steps of:
a) preparing VEGF-R protein;
b) exposing said VEGF-R protein to a test compound;
c) quantifying the binding of said compound to VEGF-R protein by any suitable means.

Utilization of the screening system described above provides a means to determine compounds that may alter the biological function of VEGF-R. This screening method may be adapted to large-scale, automated procedures such as a PANDEX® (Baxter-Dade Diagnostics) system, allowing for efficient high-volume screening of potential therapeutic agents.

In such a screening protocol VEGF-R is prepared as described herein, preferably using recombinant DNA technology. A test compound is introduced into a reaction vessel containing the VEGF-R protein or fragment thereof. Binding of VEGF-R by a test compound is determined by any suitable means. For example, in one method radioactively-labeled or chemically-labeled test compound may be used. Binding of the protein by the compound is assessed, for example, by quantifying bound label versus unbound label using any suitable method. Binding of a test compound may also be carried out by a method disclosed in U.S. Patent 5,585,277, which hereby is incorporated by reference. In this method, binding of a test compound to a protein is assessed by monitoring the ratio of folded protein to unfolded protein, for example by monitoring sensitivity of said protein to a protease, or amenability to binding of said protein by a specific antibody against the folded state of the protein.

The foregoing screening methods are useful for identifying a ligand, for example, an antagonist of a VEGF-R protein, as a lead to a pharmaceutical compound for the treatment of cancer, or for inhibiting tumor growth. A ligand that binds VEGF-R, or related fragment thereof, is identified, for example, by combining a test ligand with VEGF-R under conditions that cause the protein to exist in a ratio of folded to unfolded states. If the test ligand binds the folded state of the protein, the relative amount of folded protein will be higher than in the case of a test ligand that does not bind the protein. The ratio of protein in the folded versus unfolded state is easily determinable by, for example, susceptibility to digestion by a protease, or binding to a specific antibody, or binding to chaperonin protein, or binding to any suitable surface.

### VEGF-R Protein Therapeutic Applications

In one embodiment, the present invention relates to therapeutic applications in which angiogenesis or the inhibition thereof is therapeutically beneficial. For example, VEGF-R may be administered to enhance wound healing such as in post-surgical procedures, or in the treatment of cardiac vessel blockage, or other condition in which enhanced blood vessel formation is beneficial.

The present invention also provides methods for treating cancer and for inhibiting tumor growth, in vitro or in vivo, comprising administration of an effective amount of an VEGF-R antagonist.

For therapeutic use in wound healing or other use in which enhanced angiogenesis is advantageous, an effective amount of VEGF-R protein is administered to an organism in need thereof in a dose between about 0.1 and 1000 ug/kg body weight. In practicing the methods contemplated, VEGF-R can be administered in a single daily dose or in multiple doses per day. The amount per administration will be determined by the physician and depend on such factors as the nature and severity of the disease, and the age and general health of the patient.

The present invention also provides a pharmaceutical composition comprising as the active agent a VEGF-R polypeptide or fragment thereof, or a pharmaceutically acceptable non-toxic salt thereof, and a pharmaceutically acceptable solid or liquid carrier. For example, compounds comprising VEGF-R can be admixed with conventional pharmaceutical carriers and excipients, and used in the form of tablets, capsules, elixirs, suspensions, syrups, wafers, and the like. The compositions comprising VEGF-R will contain from about 0.1% to 90% by weight of the active compound, and more generally from about 10% to 30%. The compositions may contain common carriers and excipients such as corn starch or gelatin, lactose, sucrose, microcrystalline cellulose, kaolin, mannitol, dicalcium phosphate, sodium chloride, and alginic acid. The compounds can be formulated for oral or parenteral administration.

For intravenous (IV) use, the VEGF-R protein is administered in commonly used intravenous fluid(s) and administered by infusion. Such fluids, for example, physiological saline, Ringer's solution or 5% dextrose solution can be used.

For intramuscular preparations, a sterile formulation, preferably a suitable soluble salt form of the VEGF-R protein, for example SEQ ID NO:2, such as the hydrochloride salt, can be dissolved and administered in a pharmaceutical diluent such as pyrogen-free water (distilled), physiological saline or 5% glucose solution. A suitable insoluble form of the compound may be prepared and administered as a suspension in an aqueous base or a pharmaceutically acceptable oil base, e.g. an ester of a long chain fatty acid such as ethyl oleate.

The present invention also provides a pharmaceutical composition comprising as the active agent an antagonist of VEGF-R, or a pharmaceutically acceptable non-toxic salt thereof, and a pharmaceutically acceptable solid or liquid carrier. For example, compounds comprising VEGF-R antagonist can be admixed with conventional pharmaceutical carriers and excipients, and used in the form of tablets, capsules, elixirs, suspensions, syrups, wafers, and the like. The compositions comprising VEGF-R will contain from about 0.1% to 90% by weight of the active compound, and more generally from about 10% to 30%. The compositions may contain common carriers and excipients such as corn starch or gelatin, lactose, sucrose, microcrystalline cellulose, kaolin, mannitol, dicalcium phosphate, sodium chloride, and alginic acid. The compounds can be formulated for oral or parenteral administration.

Skilled artisans will recognize that IC₅₀ values are dependent on the selectivity of the compound tested. For example, a compound with an IC₅₀ which is less than 10 nM is generally considered an excellent candidate for drug therapy. However, a compound which has a lower affinity, but is selective for a particular target, may be an even better candidate. The skilled artisan will recognize that any information regarding the binding potential, inhibitory activity, or selectivity of a particular compound is useful toward the development of pharmaceutical products.

The following examples more fully describe the present invention. Those skilled in the art will recognize that the particular reagents, equipment, and procedures described are merely illustrative and are not intended to limit the present invention in any manner.

### EXAMPLE 1

### RT-PCR Amplification of VEGF-R Gene from mRNA

A VEGF-R gene is isolated by reverse transcriptase PCR (RT-PCR) using conventional methods. Total RNA from a tissue that expresses the VEGF-R gene, is prepared using standard methods. First strand cDNA synthesis is achieved using a commercially available kit (SuperScript™ System; Life Technologies) in conjunction with specific primers directed at any suitable region of SEQ ID NO:1, for example between residues 276 and 1310.

Amplification is carried out by adding to the first strand cDNA (dried under vacuum): 8 µl of 10X synthesis buffer (200 mM Tris-HCl, pH 8.4; 500 mM KCl, 25 mM MgCl₂, 1 ug/ul BSA); 68 µl distilled water; 1 µl each of a 10 uM solution of each primer; and 1 µl Taq DNA polymerase (2 to 5 U/µl). The reaction is heated at 94° C for 5 min. to denature the RNA/cDNA hybrid. Then, 15 to 30 cycles of PCR amplification are performed using any suitable thermal cycle apparatus. The amplified sample may be analyzed by agarose gel electrophoresis to check for an appropriately-sized fragment.

### EXAMPLE 2

### Production of a Vector for Expressing VEGF-R in a Host Cell

An expression vector suitable for expressing VEGF-R or fragment thereof in a variety of procaryotic host cells, such as *E. coli* is easily made. The vector contains an origin of replication (Ori), an ampicillin resistance gene (Amp) useful for selecting cells which have incorporated the vector following a tranformation procedure, and further comprises the T7 promoter and T7 terminator sequences in operable linkage to a VEGF-R coding region. Plasmid pET11A (obtained from Novogen, Madison WI) is a suitable parent plasmid. pET11A is linearized by restriction with endonucleases NdeI and BamHI. Linearized pET11A is ligated to a DNA fragment bearing NdeI and BamHI sticky ends and comprising the coding region of the VEGF-R gene as disclosed by SEQ ID NO:1 viz. Residues 276 through 1310, or fragment thereof.

The VEGF-R gene used in this construction may be slightly modified at the 5' end (amino terminus of encoded protein) in order to simplify purification of the encoded protein product. For this purpose, an oligonucleotide encoding 8 histidine residues is inserted after the ATG start codon. Placement of the histidine residues at the amino terminus of the encoded protein serves to enable the IMAC one-step protein purification procedure.

### EXAMPLE 3

### Recombinant Expression of Modified VEGF-R Protein

An expression vector that carries an ORF encoding residues 16 though 345 of SEQ ID NO:2 and further comprising a kappa chain signal peptide fused at the amino terminus of said residue 16 was operably-linked to an CMV promoter in plasmid pcDNA3 and transfected into 293 T cells using standard methods. Transfectants were analyzed for transient expression of VEGF-R using Western blot analysis. The results showed that the truncated VEGF-R protein was secreted into the culture medium.

### EXAMPLE 4

### Detecting Ligands that Bind VEGF-R Using a Chaperonin Protein Assay

The wells of an ELISA plate are coated with chaperonin by incubation for several hours with a 4 ug/ml solution of the protein in Tris-buffered Saline (TBS: 10 mM Tris-HCl, pH7.5, 0.2M NaCl). The plates are then washed 3 times with TBS containing 0.1% Tween-20 (TBST). Then, a mixture of VEGF-R protein (sufficient amount to saturate about 50% of the binding sites on chaperonin) and test compound (10⁻⁹ to 10⁻⁵ M) in about 50 µl volume is added to each well of the plate for an incubation of about 60 minutes. Aliquots of the well solutions are then transferred to the wells of fresh plates and incubated for 60 minutes at room temperature, followed by 3 washes with TBST. Next, about 50 µl of an antibody specific for VEGF-R plus 5% nonfat dry milk are added to each well for a 30 minute incubation at room temperature. After washing, about 50 µl of goat anti-rabbit IgG alkaline phosphatase conjugate at an appropriate dilution in TBST plus 5% nonfat dry milk are added to each will and incubated 30 minutes at room temperature. The plates are washed again with TBST and 0.1 ml of 1 mg/ml p-nitrophenylphosphate in 0.1% diethanolamine is added. Color development (proportional to bound alkaline phosphatase antibody conjugate) is monitored with an ELISA plate reader. When test ligand binding has occurred, ELISA analysis reveals VEGF-R in solution at higher concentrations than in the absence of test ligand.

### EXAMPLE 5

### Production of an Antibody to VEGF-R Protein

Substantially pure VEGF-R protein or fragment thereof is isolated from transfected or transformed cells using any of the well known methods in the art, or by a method specifically disclosed herein. Concentration of protein in a final preparation is adjusted, for example, by filtration through an Amicon filter device such that the level is about 1 to 5 ug/ml. Monoclonal or polyclonal antibody can be prepared as follows.

Monoclonal antibody can be prepared from murine hybridomas according to the method of Kohler and Milstein (*Nature*, 256, 495, 1975), or a modified method thereof. Briefly, a mouse is repetitively inoculated with a few micrograms of the protein or fragment thereof, or fusion peptide thereof, over a period of a few weeks. The mouse is then sacrificed and the antibody producing cells of the spleen isolated. The spleen cells are fused by means of polyethylene glycol with mouse myeloma cells. Fused cells that produce antibody are identified by any suitable immunoassay, for example, ELISA, as described in E. Engvall, *Meth. Enzymol.*, 70, 419, 1980.

Polyclonal antiserum can be prepared by well known methods (*See e.g.* J. Vaitukaitis *et.al. Clin. Endocirnol. Metab.* 33, 988, 1971) that involve immunizing suitable animals with the proteins, fragments thereof, or fusion proteins thereof, disclosed herein. Small doses (e.g. nanogram amounts) of antigen administered at multiple intradermal sites appears to be the most reliable method.

### EXAMPLE 6

### Endothelial Cell Growth Assay

A recombinant plasmid that expresses VEGF-R, or functionally related fragment, is transfected by calcium phosphate precipitation into human embryonic kidney cell line 293, cultured in Dulbecco's Modified Eagle's Medium (DMEM)F-12 (1:1) containing 2 mM glutamine and 10% fetal bovine serum. After overnight incubation with the calcium phosphate-DNA mixture, the medium is replaced with serum-free medium and incubated for an additional 48-72 hours before assaying the medium for biological activity.

For conducting the cell growth assay, bovine capillary endothelial cells are maintained in DMEM containing 20% calf serum according to the method of Ferrara, Biochem. Biophys. Res. Comm., 161, 851-58, 1989. Cells are plated at about 8 x 10³ cells per well in 12 well plates in DMEM supplemented with 10% calf serum, 2 mM glutamine, and antibiotics. Conditioned medium from transiently transfected 293 cells 72 hours post-transfection is added and cell number determined after 5 days.

### EXAMPLE 7

### Assay for VEGF-R Antagonist

Assay reactions are set up essentially as described in Example 6, except that a compound to be tested for VEGF-R antagonist activity is included at the step of adding conditioned medium to bovine capillary endothelial cells. Multiple assays can be set up in which a constant amount of conditioned medium is incubated with varying amounts of test compound, for example from about 10 ng/ml to about 100 ug/ml.

For conducting the cell growth assay, bovine capillary endothelial cells are maintained in DMEM containing 20% calf serum according to the method of Ferrara, Biochem. Biophys. Res. Comm., 161, 851-58, 1989. Cells are plated at about 8 x 10³ cells per well in 12 well plates in DMEM supplemented with 10% calf serum, 2 mM glutamine, and antibiotics. Conditioned medium from transiently transfected 293 cells 72 hours post-transfection is added and cell number determined after 5 days.

## Claims

1. A substantially pure protein having the amino acid sequence designated herein as:
a) SEQ ID NO:2,
b) an analog of SEQ ID NO:2.
c) a functional fragment of SEQ ID NO:2;
d) residues from about 16 through 345 of SEQ ID NO:2; or
e) residues from about 23 through 345 of SEQ ID NO:2.

2. An isolated nucleic acid encoding a protein of Claim 1.

3. An isolated nucleic acid as in claim 2 wherein said nucleic acid comprises SEQ ID NO:1, or nucleotide residues 276 through 1310 of SEQ ID NO:1.

4. An isolated nucleic acid that hybridizes to a nucleic acid defined by residues 276 through 1310 of SEQ ID NO:1 under high stringency conditions.

5. A vector comprising an isolated nucleic acid compound of Claims 2 through Claim 4.

6. A host cell containing a vector of Claim 5.

7. A method for constructing a recombinant host cell having the potential to express SEQ ID NO:2, said method comprising introducing into said host cell by any suitable means a vector of Claim 5.

8. A pharmaceutical formulation comprising as an active ingredient VEGF-R protein, or analog thereof, associated with one or more pharmaceutically acceptable carriers, excipients, or diluents thereof.

9. A pharmaceutical formulation comprising as an active ingredient an antagonist of an VEGF-R, associated with one or more pharmaceutically acceptable carriers, excipients, or diluents thereof.

10. A method for identifying an VEGF-R antagonist comprising the steps of:
a) admixing a substantially purified preparation of VEGF-R with a test compound; and
b) monitoring, by any suitable means, an inhibition in the biological activity of VEGF-R.


SEQUENCE LISTING

<110> Song, Ho Yeong

Na, Songqing

Dou, Shenshen

Su, Eric Wen
Wei, Jian-Jun

<120> VEGF Related Gene and Protein

<130> X-11851C

<140>

<141>

<160> 2

<170> PatentIn Ver. 2.0

<210> 1

<211> 2898

<212> DNA

<213> Homo sapiens

<220>

<221> CDS

<222> (276)..(1310)

<400> 1

cttcgccaca gctcaggatt tgtttaaacc ttgggtaaac tggttcaggt ccaggttttg 60

ctttgatcct tttcaaaaac tggatacaca gaagagggct ctaggaaaaa gttttggatg 120

ggattatgtg gaaactaccc tgcgattctc tgctgccaga gcaggctcgg cgcttccacc 180

ccagtgcagc cttcccctgg cggtggtgaa agagactcgg gagtcgctgc ttccaaagtg 240

cccgccgtga gtgagctctc accccagtca gccaa atg agc ctc ttc ggg ctt 293
Met Ser Leu Phe Gly Leu
1               5

ctc ctg ctg aca tct gcc ctg gcc ggc cag aga cag ggg act cag gcg 341
Leu Leu Leu Thr Ser Ala Leu Ala Gly Gln Arg Gln Gly Thr Gln Ala
            10                  15                  20

gaa tcc aac ctg agt agt aaa ttc cag ttt tcc agc aac aag gaa cag 389
Glu Ser Asn Leu Ser Ser Lys Phe Gln Phe Ser Ser Asn Lys Glu Gln
        25                  30                  35

aac gga gta caa gat cct cag cat gag aga att att act gtg tct act 437
Asn Gly Val Gln Asp Pro Gln His Glu Arg Ile Ile Thr Val Ser Thr
    40                  45                  50

aat gga agt att cac agc cca agg ttt cct cat act tat cca aga aat 485
Asn Gly Ser Ile His Ser Pro Arg Phe Pro His Thr Tyr Pro Arg Asn
55                  60                  65                  70

acg gtc ttg gta tgg aga tta gta gca gta gag gaa aat gta tgg ata 533
Thr Val Leu Val Trp Arg Leu Val Ala Val Glu Glu Asn Val Trp Ile
                75                  80                  85

caa ctt acg ttt gat gaa aga ttt ggg ctt gaa gac cca gaa gat gac 581

Gln Leu Thr Phe Asp Glu Arg Phe Gly Leu Glu Asp Pro Glu Asp Asp
            90                  95                  100

ata tgc aag tat gat ttt gta gaa gtt gag gaa ccc agt gat gga act 629
Ile Cys Lys Tyr Asp Phe Val Glu Val Glu Glu Pro Ser Asp Gly Thr
        105                 110                 115

ata tta ggg cgc tgg tgt ggt tct ggt act gta cca gga aaa cag att 677
Ile Leu Gly Arg Trp Cys Gly Ser Gly Thr Val Pro Gly Lys Gln Ile
    120                 125                 130

tct aaa gga aat caa att agg ata aga ttt gta tct gat gaa tat ttt 725
Ser Lys Gly Asn Gln Ile Arg Ile Arg Phe Val Ser Asp Glu Tyr Phe
135                 140                 145                 150

cct tct gaa cca ggg ttc tgc atc cac tac aac att gtc atg cca caa 773
Pro Ser Glu Pro Gly Phe Cys Ile His Tyr Asn Ile Val Met Pro Gln
                155                 160                 165

ttc aca gaa gct gtg agt cct tca gtg cta ccc cct tca gct ttg cca 821
Phe Thr Glu Ala Val Ser Pro Ser Val Leu Pro Pro Ser Ala Leu Pro
            170                 175                 180

ctg gac ctg ctt aat aat gct ata act gcc ttt agt acc ttg gaa gac 869
Leu Asp Leu Leu Asn Asn Ala Ile Thr Ala Phe Ser Thr Leu Glu Asp
        185                 190                 195

ctt att cga tat ctt gaa cca gag aga tgg cag ttg gac tta gaa gat 917
Leu Ile Arg Tyr Leu Glu Pro Glu Arg Trp Gln Leu Asp Leu Glu Asp
    200                 205                 210

cta tat agg cca act tgg caa ctt ctt ggc aag gct ttt gtt ttt gga 965
Leu Tyr Arg Pro Thr Trp Gln Leu Leu Gly Lys Ala Phe Val Phe Gly
215                 220                 225                 230

aga aaa tcc aga gtg gtg gat ctg aac ctt cta aca gag gag gta aga 1013
Arg Lys Ser Arg Val Val Asp Leu Asn Leu Leu Thr Glu Glu Val Arg
                235                 240                 245

tta tac agc tgc aca cct cgt aac ttc tca gtg tcc ata agg gaa gaa 1061
Leu Tyr Ser Cys Thr Pro Arg Asn Phe Ser Val Ser Ile Arg Glu Glu
            250                 255                 260

cta aag aga acc gat acc att ttc tgg cca ggt tgt ctc ctg gtt aaa 1109
Leu Lys Arg Thr Asp Thr Ile Phe Trp Pro Gly Cys Leu Leu Val Lys
        265                 270                 275

cgc tgt ggt ggg aac tgt gcc tgt tgt ctc cac aat tgc aat gaa tgt 1157
Arg Cys Gly Gly Asn Cys Ala Cys Cys Leu His Asn Cys Asn Glu Cys
    280                 285                 290

caa tgt gtc cca agt aaa gtt act aaa aaa tac cac gag gtc ctt cag 1205
Gln Cys Val Pro Ser Lys Val Thr Lys Lys Tyr His Glu Val Leu Gln
295                 300                 305                 310

ttg aga cca aag acc ggt gtc agg gga ttg cac aaa tca ctc acc gac 1253
Leu Arg Pro Lys Thr Gly Val Arg Gly Leu His Lys Ser Leu Thr Asp
                315                 320                 325

gtg gcc ctg gag cac cat gag gag tgt gac tgt gtg tgc aga ggg agc 1301
Val Ala Leu Glu His His Glu Glu Cys Asp Cys Val Cys Arg Gly Ser

            330                 335                 340

aca gga gga tagccgcatc accaccagca gctcttgccc agagctgtgc 1350
Thr Gly Gly
        345

agtgcagtgg ctgattctat tagagaacgt atgcgttatc tccatcctta atctcagttg 1410

tttgcttcaa ggacctttca tcttcaggat ttacagtgca ctctgaaaga ggagacatca 1470

aacagaatta ggagttgtgc aacagctctt ttgagaggag gcctaaagga caggagaaaa 1530

ggtcttcaat cgtggaaaga aaattaaatg ttgtattaaa tagatcacca gctagtttca 1590

gagttaccat gtacgtattc cactagctgg gttctgtatt tcagttcttt cgatacggct 1650

tagggtaatg tcagtacagg aaaaaactgt gcaagtgagc acctgattcc gttgccttgc 1710

ttaactctaa agctccatgt cctgggccta aaatcgtata aaatctggat tttttttttt 1770

ttttttgctc atattcacat atgtaaacca gaacattcta tgtactacaa acctggtttt 1830

taaaaaggaa ctatgttgct atgaattaaa cttgtgtcat gctgatagga caggctggat 1890

ttttcatatt tcttattaaa atttctgcca tttagaagaa gagaactaca ttcatggttt 1950

ggaagagata aacctgaaaa gaagagtggc cttatcttca ctttatcgat aagtcagttt 2010

atttgtttca ttgtgtacat ttttatattc tccttttgac attataactg ttggcttttc 2070

taatcttgtt aaatatatct atttttacca aaggtattta atattctttt ttatgacaac 2130

ttagatcaac tatttttagc ttggtaaatt tttctaaaca caattgttat agccagagga 2190

acaaagatga tataaaatat tgttgctctg acaaaaatac atgtatttca ttctcgtatg 2250

gtgctagagt tagattaatc tgcattttaa aaaactgaat tggaatagaa ttggtaagtt 2310

gcaaagactt tttgaaaata attaaattat catatcttcc attcctgtta ttggagatga 2370

aaataaaaag caacttatga aagtagacat tcagatccag ccattactaa cctattcctt 2430

tttttgggga aatctgagcc tagctcagaa aaacataaag caccttgaaa aagacttggc 2490

agcttcctga taaagcgtgc tgtgctgtgc agtaggaaca catcctattt attgtgatgt 2550

tgtggtttta ttatcttaaa ctctgttcca tacacttgta taaatacatg gatattttta 2610

tgtacagaag tatgtctctt aaccagttca cttattgtac tcaggcaatt taaaagaaaa 2670

tcagtaaaat attttgcttg taaaatgctt aatatcgtgc ctaggttatg tggtgactat 2730

ttgaatcaaa aatgtattga atcatcaaat aaaagaatgt ggctattttg gggagaaaag 2790

taaaaaaaaa aaaaaaaaaa aaaaaaaaaa aaaaaaagcg gncgctgaat tacctgcccg 2850

ggcgggcgct ccaaccctat agtgagtnag ccgaattcca gcacactg 2898

<210> 2

<211> 345

<212> PRT

<213> Homo sapiens

<400> 2

Met Ser Leu Phe Gly Leu Leu Leu Leu Thr Ser Ala Leu Ala Gly Gln
1               5                   10                  15

Arg Gln Gly Thr Gln Ala Glu Ser Asn Leu Ser Ser Lys Phe Gln Phe
            20                  25                  30

Ser Ser Asn Lys Glu Gln Asn Gly Val Gln Asp Pro Gln His Glu Arg
        35                  40                  45

Ile Ile Thr Val Ser Thr Asn Gly Ser Ile His Ser Pro Arg Phe Pro
    50                  55                  60

His Thr Tyr Pro Arg Asn Thr Val Leu Val Trp Arg Leu Val Ala Val
65                  70                  75                  80

Glu Glu Asn Val Trp Ile Gln Leu Thr Phe Asp Glu Arg Phe Gly Leu
                85                  90                  95

Glu Asp Pro Glu Asp Asp Ile Cys Lys Tyr Asp Phe Val Glu Val Glu
            100                 105                 110

Glu Pro Ser Asp Gly Thr Ile Leu Gly Arg Trp Cys Gly Ser Gly Thr
        115                 120                 125

Val Pro Gly Lys Gln Ile Ser Lys Gly Asn Gln Ile Arg Ile Arg Phe
    130                 135                 140

Val Ser Asp Glu Tyr Phe Pro Ser Glu Pro Gly Phe Cys Ile His Tyr
145                 150                 155                 160

Asn Ile Val Met Pro Gln Phe Thr Glu Ala Val Ser Pro Ser Val Leu
                165                 170                 175

Pro Pro Ser Ala Leu Pro Leu Asp Leu Leu Asn Asn Ala Ile Thr Ala
            180                 185                 190

Phe Ser Thr Leu Glu Asp Leu Ile Arg Tyr Leu Glu Pro Glu Arg Trp
        195                 200                 205

Gln Leu Asp Leu Glu Asp Leu Tyr Arg Pro Thr Trp Gln Leu Leu Gly
    210                 215                 220

Lys Ala Phe Val Phe Gly Arg Lys Ser Arg Val Val Asp Leu Asn Leu
225                 230                 235                 240

Leu Thr Glu Glu Val Arg Leu Tyr Ser Cys Thr Pro Arg Asn Phe Ser
                245                 250                 255

Val Ser Ile Arg Glu Glu Leu Lys Arg Thr Asp Thr Ile Phe Trp Pro
            260                 265                 270

Gly Cys Leu Leu Val Lys Arg Cys Gly Gly Asn Cys Ala Cys Cys Leu
        275                 280                 285

His Asn Cys Asn Glu Cys Gln Cys Val Pro Ser Lys Val Thr Lys Lys
    290                 295                 300

Tyr His Glu Val Leu Gln Leu Arg Pro Lys Thr Gly Val Arg Gly Leu
305                 310                 315                 320

His Lys Ser Leu Thr Asp Val Ala Leu Glu His His Glu Glu Cys Asp
                325                 330                 335

Cys Val Cys Arg Gly Ser Thr Gly Gly
            340                 345